(19)

Europäisches Patentamt
European Patent Office
Office européen des brevets

(11) **EP 4 692 795 A1**

(12) **EUROPEAN PATENT APPLICATION**
published in accordance with Art. 153(4) EPC

(43) Date of publication:
**11.02.2026 Bulletin 2026/07**

(21) Application number: **24777928.3**

(22) Date of filing: **22.03.2024**

(51) International Patent Classification (IPC):
*G01N 33/543* (2006.01)  *G01N 11/00* (2006.01)
*C12M 1/42* (2006.01)  *G01N 21/55* (2014.01)

(52) Cooperative Patent Classification (CPC):
**C12M 1/00; C12M 1/34; C12M 1/42; C12Q 1/02;
G01N 11/00; G01N 21/55; G01N 33/543;
G06V 20/69**

(86) International application number:
**PCT/CN2024/083360**

(87) International publication number:
**WO 2024/199139 (03.10.2024 Gazette 2024/40)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: 24.03.2023  CN 202310299296
24.03.2023  CN 202310299292
24.03.2023  CN 202310299290
24.03.2023  CN 202310299306
24.03.2023  CN 202310299309
24.03.2023  CN 202310297791
24.03.2023  CN 202310299308

(71) Applicants:
• **Yigong Ruishi (Fujian) Engineering Research
Center Co., Ltd.
Fuzhou, Fujian 350109 (CN)**
• **Yigong Ruixin (Xiamen) Technology Co., Ltd
Xiamen, Fujian 361015 (CN)**

(72) Inventor: **LIN, Zhe
Xiamen, Fujian 361015 (CN)**

(74) Representative: **Chung, Hoi Kan
Mandarin IP Limited
7 Cherry Trees
Great Shelford
Cambridge CB22 5XA (GB)**

(54) **MULTI-MODAL FLUID CHARACTERIZATION AND DISTURBANCE SYSTEM AND METHOD,
AND APPLICATION**

(57)    The present invention relates to the field of fluid measurement, especially a fluid characterization and perturbation system, method, and application thereof. It includes a base, and a micropillar array arranged on the base, the micropillar array being composed of at least one micropillar, the micropillars being capable of deforming under the action of a fluid and/or a magnetic force, and the length and hardness of different micropillars can be different; the micropillar includes a bottom end connected to the base, a side surface, and a top end opposite the bottom end and away from the base, the micropillar being provided with a light-reflecting layer, the light-reflecting layer being deployable at at least one arbitrary position on the top end, side surface, or within the pillar body of the micropillar. The system and method of the present invention have multimodal characteristics, can comprehensively measure data such as the viscosity, density, and type of a fluid, as well as the fluid direction, pressure, and shear force at specific locations, providing a comprehensive solution for the monitoring and analysis of fluid states.

Example of base and micropillar arrangement

Figure 5

## Description

## TECHNICAL FIELD

The present invention relates to the field of fluid measurement, especially a fluid characterization and perturbation system, method, and application thereof.

## BACKGROUND ART

Fluid mechanics is the science that studies the motion of fluids and their interaction with solids. Fluid mechanics has wide applications in fields such as engineering, physics, chemistry, and biology. In order to gain a deep understanding of the physical properties and dynamic behavior of fluids, it is necessary to perform precise characterization and control of the flow field. The characteristics of fluid flow velocity, etc., in microfluidic channels are of great significance for inferring the state of the device and the reaction. Traditional flow field characterization methods mainly include optical methods, electromagnetic methods, acoustic wave methods, etc. For example, methods for measuring fluid flow velocity include Pitot tube velocimetry, but it greatly disturbs the flow field; hot-wire anemometry, but its probe greatly disturbs the flow field and the hot wire is easily broken; Laser Doppler Velocimetry (LDV) and Particle Image Velocimetry (PIV), but the systems are complex and rely on bubbles or tracer particles in the fluid. All of the above methods are difficult to miniaturize to be embedded into microfluidic channels. Moreover, they usually require expensive instrumentation paired with data processing algorithms, and it is difficult to achieve high-resolution and high-sensitivity measurements of the flow field at the micro- and nano-scales. Therefore, developing a simple, effective, and multifunctional flow field characterization and perturbation device has important scientific significance and practical value.

## SUMMARY OF THE INVENTION

For this purpose, it is necessary to provide a fluid characterization and perturbation system that can overcome the prior art, being multimodal, real-time, and low-cost without requiring expensive equipment, to meet the needs for tools and methods in microfluidics, micro-control, microreactors, chemical engineering, and biological fluid characterization.
To achieve the above objective, in a first aspect, the present invention provides a multimodal fluid characterization and perturbation apparatus, comprising:

a base, whose morphology can be a plane, an inclined surface, a curved surface, or an irregular surface, and

a micropillar array arranged on the base, the micropillar array being composed of at least one micropillar, these micropillar being capable of undergoing a state change under the action of a fluid and/or a magnetic force, and the length and hardness of different micropillars can be different;

the state change comprising at least one of: pulling, movement in various directions, and deformation;

the micropillar comprising a bottom end connected to the base, a side surface, and a top end opposite the bottom end and away from the base, the micropillar being provided with a light-reflecting layer, the light-reflecting layer being deployed at least at one position of the top end, side surface, and within the pillar body of the micropillar.

The term "several" in the present application does not mean a plural quantity, but refers to one or more.
Furthermore, an anti-reflection layer is provided on the side surface of the micropillar and/or the surface of the base. This arrangement can significantly improve the signal-to-noise ratio of the light reflection signal, making the measurement results more accurate.
Furthermore, the light-reflecting layer of the multimodal fluid characterization and perturbation apparatus is selected from at least one of inorganic reflective materials such as metals, metal oxides, metal salts, ultrafine glass beads, microprisms, and organic reflective materials. The above-listed materials for the light-reflecting layer can be used alone or in combination.
Furthermore, a magnetic substance is deployed at least at one position of the top end, side surface, and within the pillar body of the micropillar on the multimodal fluid characterization and perturbation apparatus, and preferably, a magnetic metal light-reflecting layer is deployed on the top end of the micropillar.
The magnetic substance in the present application refers to materials that react in some way to a magnetic field, including but not limited to ferromagnetic materials, paramagnetic materials, diamagnetic materials, ferrimagnetic substances, antiferromagnetic substances, and superparamagnetic materials.
Furthermore, the multimodal fluid characterization and perturbation apparatus further comprises several flow field confinement structures arranged on the base, the flow field confinement structure comprising one or several confinement surfaces, the confinement surface being a plane or a curved surface that is perpendicular to the plane where the base is located, and connected to the base or integrally formed with the base.

Furthermore, the base and/or micropillars are made of a conductive material. This arrangement, in addition to being able to apply electrical stimulation to the fluid, can also achieve the purpose of receiving electrical signals from the fluid.

Furthermore, the cross-section of the micropillar can be of any shape, including a circle, an ellipse, or a polygon. By utilizing the characteristic of a specific shape, such as an ellipse, being more easily deformable in the direction of the short axis and less easily deformable in the direction of the long axis, an array composed of specifically shaped micropillars can be used to sense the direction of the flow field.

Furthermore, a fluorescent substance is provided on the extension part of the micropillar from the top end to the bottom end. The extension part includes the side surface of the micropillar and the solid part of the pillar body enclosed by the side surface. This arrangement can achieve the purpose of more intuitively calibrating the system to amplify the signal, improve the signal-to-noise ratio, and enhance specificity. At the same time, combining it with an optical waveguide can better achieve miniaturization.

Furthermore, the micropillars can be deployed within a channel to be inserted into the flow field, or can be deployed below the channel with their top ends flush with the channel boundary, to measure the wall friction resistance, $t_{wall}$.

Specifically, the multicellular aggregate of the present application is combined with the multimodal fluid characterization and perturbation apparatus in various ways, two specific ways of which are as follows:

The first way of combination: a culture medium is placed on the micropillars of the multimodal fluid characterization and perturbation apparatus, and cells are transplanted into the culture medium on the micropillars to be cultured into a multicellular aggregate. In some other embodiments, with this way of combination, under the condition that cell mechanical force information is output in a visual form, the cultivation process of the cells can be monitored in real-time, which can be applied to study the effects of chemical, biological, and physical external stimuli on cell growth, such as culture media, drugs, etc.

The second way of combination: the already cultured multicellular aggregate is directly adhered to the micropillars of the multimodal fluid characterization and perturbation apparatus for detection.

To achieve the above objective, in a second aspect, the present invention provides a multimodal fluid characterization and perturbation characterization system.

It comprises:

at least one multimodal fluid characterization and perturbation apparatuses according to the first aspect of the present invention;

a light signal emitting device, for emitting a predetermined light beam;

a light signal detecting device, for detecting the light beam reflected from the light-reflecting layer;

a magnetic field emitting device, for emitting a magnetic field, to generate a magnetic force with the magnetic material (if any) contained at any position on the top end, side edge, or within the pillar body of the micropillar;

wherein the light beam emitted by the light signal emitting device illuminates the light-reflecting layer through an incident light path, and the light beam reflected by the light-reflecting layer enters the light signal detecting device through a reflected light path.

Furthermore, the multimodal fluid characterization and perturbation also comprises a light signal analysis device, the light signal analysis device being used to analyze the light signal.

Furthermore, the multimodal fluid characterization and perturbation system further comprises a data processing device, the data processing device being used to compute and process the light signal to obtain a flow field state (including the flow field shear force, flow field direction, flow velocity, viscous force, flow state, etc., at a certain specific location) result; and/or, directly obtaining the flow field state result from the light signal analyzed by the light signal analysis device.

Furthermore, the data processing device can also assess the chemical, physical, and biological states within the system based on the flow field state, and provide predictive reaction information.

Furthermore, the multimodal fluid characterization and perturbation system further comprises a pressure sensing device, the pressure sensing device being used to measure pressure information and/or apply appropriate pressure to the fluid.

Furthermore, the light signal emitting device has a light source, the light source comprising a first light source arranged at the bottom end of the base and/or a second light source arranged at the side surface of the base, wherein the light beam emitted by the first light source and/or second light source reaches the micropillars.

In the present application, the first light source and second light source do not imply a limitation on the setting order and type of light sources, but are merely for distinguishing light sources at different set positions. Preferably, the second light source employs a waveguide illumination source.

Furthermore, the multimodal fluid characterization and perturbation system further comprises a motion or deformation device for applying an appropriate mechanical force to cause motion or deformation, the motion or deformation device for applying an appropriate mechanical force being used to apply a mechanical force to the multimodal fluid characterization and perturbation system. the motion or deformation device for applying an appropriate mechanical force includes but is not limited to a pulling device or an inflation/deflation device, the pulling device being used to apply an appropriate mechanical pulling force to the base to control the stretching motion and/or deformation of the base in the horizontal direction, and the inflation/deflation device being used to form a motion or deformation on a curved surface of the base.

Furthermore, the multimodal fluid characterization and perturbation system is constituted by 2 or more multimodal fluid characterization and perturbation apparatuses to form a double-sided structure or a multi-sided structure, wherein the outside of the double-sided structure or multi-sided structure is the base, and the inside encloses a three-dimensional containment chamber for the fluid;

the base of each multimodal fluid characterization and perturbation apparatus forms a surface of the three-dimensional containment chamber; when forming the three-dimensional containment chamber, at least one surface of the three-dimensional containment chamber is connected with micropillars, and other surfaces of the three-dimensional containment chamber may optionally not be provided with micropillars.

Furthermore, when a double-sided or multi-sided structure is constituted by 2 or more of the multimodal fluid characterization and perturbation apparatuses, the fluid confinement structures are adapted to each other.

In a third aspect, the present invention provides a method for performing fluid characterization using the multimodal fluid characterization and perturbation system according to the second aspect of the present invention, comprising the following steps:

the multimodal fluid characterization and perturbation apparatus emitting a predetermined light beam using the light signal emitting device;

detecting the light beam after it has interacted with the multimodal fluid characterization and perturbation apparatus using the light signal detecting device. Furthermore, the method further comprises the step of emitting a magnetic field of a predetermined direction and intensity with the magnetic field emitting device.

Furthermore, the method further comprises a light signal analysis step: using the light signal analysis device to perform a comparative analysis of the reflected light beams before and after the fluid to be characterized passes through the multimodal fluid characterization and perturbation apparatus, and/or, using the light signal analysis device to perform a comparative analysis of the reflected light beams before and after the magnetic field emitting device emits a magnetic field of a predetermined direction and intensity, to obtain fluid information, the fluid information comprising the flow field shear force, flow field direction, flow velocity, viscous force, and flow state at a certain specific location, and the dynamic changes over time of the spatial distribution of the fluid information.

In a fourth aspect, the present application provides the applications of the multimodal fluid characterization and perturbation apparatus as described in the first aspect in microfluidics, micro-control, microreactors, chemical engineering, and biological fluids. These applications include, but are not limited to, characterizing the fluid within microfluidic and micro-control systems through optical signals, monitoring the situation of biological, chemical, and physical reactions within microreactors and making predictions, and characterizing biological fluids such as blood and integrating them into organ-on-a-chip systems.

Distinguished from the prior art, the above technical solution has the following advantages:

(1) Multimodal characteristics: The present invention has multimodal characteristics, can comprehensively measure data such as the viscosity, density, and type of a fluid, as well as the fluid direction, pressure, and shear force at specific locations, providing a comprehensive solution for the monitoring and analysis of fluid states.

(2) Intuitive and sensitive: The present invention converts fluid state parameters into an intuitively readable light attenuation signal, facilitating real-time monitoring and data analysis, while also simplifying the data processing and interpretation process. The deformation of the micropillars is also amplified while being converted into an optical signal, ensuring a good signal-to-noise ratio and sensitivity.

(3) Simple fabrication, low cost: The fabrication process of the present invention is simple, and the cost is relatively low. It does not require complex equipment to perform comprehensive characterization and perturbation of fluids, which is conducive to popularization, application, and industrialization.

(4) Compact and portable: The present invention has the advantages of being compact in size, allowing for precise characterization of different positions and different layers within a fluid, making it suitable for various application scenarios, especially for easy integration into microfluidic and microreactor systems, greatly expanding the application space.

(5) Multifunctional perturbation operation: In addition to monitoring the fluid state, the present invention can also perform perturbation operations such as stirring and mixing on the fluid through the magnetic field and pressure sensing system, meeting the needs of different experimental and production processes.

(6) Wide measurement range: By dynamically changing the softness/hardness of the micropillars in real-time with a magnetic field, combined with micropillars of different lengths and hardness, the present invention greatly expands the measurable range, making the monitoring of various fluid states more accurate and flexible.

**BRIEF DESCRIPTION OF THE DRAWINGS**

Figure 1 is a schematic diagram of the micropillar structure and the magnetic field-driven motion mode in a multimodal fluid characterization apparatus;

Figure 2 is a schematic diagram of the deployment of a magnetic material and a light-reflecting layer;

Figure 3 is a schematic diagram of the deployment of a fluorescent substance and a light-reflecting layer;

Figure 4 is a schematic diagram of fabrication method, an electron microscope characterization image of devices with different interfaces, lengths, and aspect ratios, an electron microscope characterization image of a metal coating, and a schematic diagram of the calculation principle;

Figure 5 is a schematic diagram of the deployment of different bases and micropillars;

Figure 6 is a schematic diagram of a multimodal fluid characterization system;

Figure 7 is a schematic diagram of measuring fluid direction with an elliptical interface micropillar;

Figure 8 is a schematic diagram of the deployment of a double-sided and a multi-sided structure;

Figure 9 is a schematic diagram of an integrated pressure sensor and a fluid perturbation device;

Figure 10 is a schematic diagram of a confinement surface and a multi-sensor combination;

Figure 11 is a schematic diagram of deploying sensors at different positions;

Figure 12 is a schematic diagram of measuring a flow field and performing system calibration using the reflected light from a micropillar sensor.

**DETAILED DESCRIPTION OF EMBODIMENTS**

To better explain the present invention for ease of understanding, the following provides a detailed description of the present invention through specific embodiments.

**Embodiment 1**

**A Multimodal Fluid Characterization Apparatus, System, and Method**

This embodiment provides a multimodal fluid characterization and perturbation apparatus that utilizes a light-reflecting layer, a magnetic material, or a magnetic metal light-reflecting layer to reflect an optical signal. The intensity of the light reflection signal is linearly related, within a certain range, to the deformation caused by the force applied on the micropillars in the fluid, thereby achieving the characterization of the fluid.

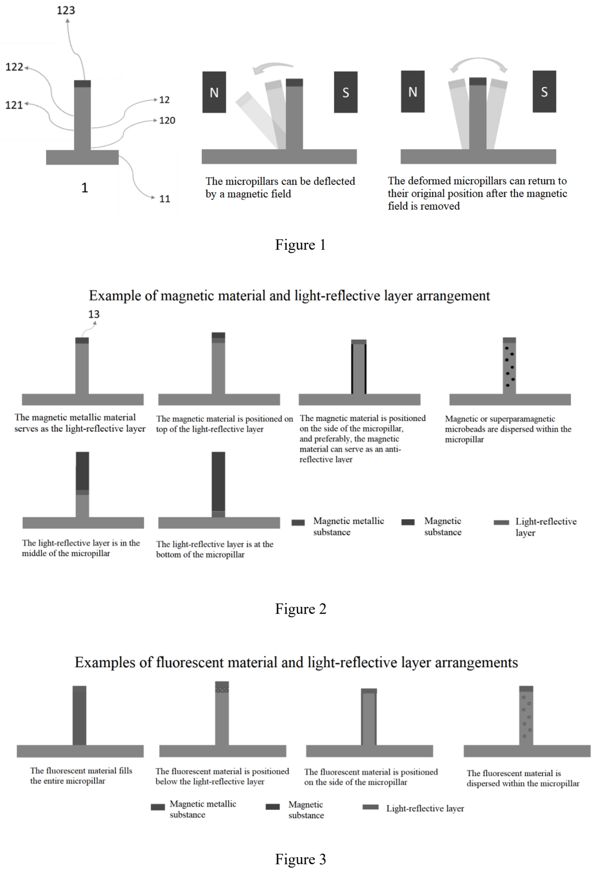

Please refer to Figure 1, which shows a multimodal fluid characterization and perturbation apparatus 1, being composed of a base 11 and multiple micropillars 12 arranged on the base 11 to form a micropillar array, thus constituting the multimodal fluid characterization apparatus 1, i.e., a micropillar chip. The micropillar 12 is composed of a bottom end 120 connected to the base 11, a side surface 121, a pillar body 122 enclosed by the side surface, and a top end 123 opposite the bottom end 120 and away from the base 11. The top end 123 of the micropillar 12 has a coating that reflects light (Figure 2); or, the side surface 121 of the micropillar 12 has a coating that reflects light (Figure 2); or, both the top end 123 and the side surface 121 of the micropillar 12 have a coating that reflects light (Figure 2). The top end 123 of the micropillar 12 has a coating of a magnetic metal (such as iron, cobalt, nickel, etc.) (in other embodiments, other magnetic materials can be used instead, and the position where the magnetic material is set can be the side surface or within the pillar body of the micropillar, as long as the micropillar can generate a magnetic force under a magnetic field). This coating with the magnetic metal 131 can serve as the light-reflecting layer 13. In a preferred embodiment, what is provided on the top end 123 of the micropillar 12 is a magnetic metal light-reflecting layer 13 (Figure 2). Preferably, a fluorescent substance can be deployed anywhere from the top end of the micropillar to the base (Figure 3), to aid in imaging to assist with system calibration or to enhance the signal-to-noise ratio.

It should be noted that the term "coating" is used in this embodiment only to indicate that the light-reflecting layer 13 in this embodiment can be prepared by a coating process, but this does not limit the light-reflecting layer 13 to being prepared only by a coating process; it can also be prepared by processes such as sputtering to create the reflective layer.

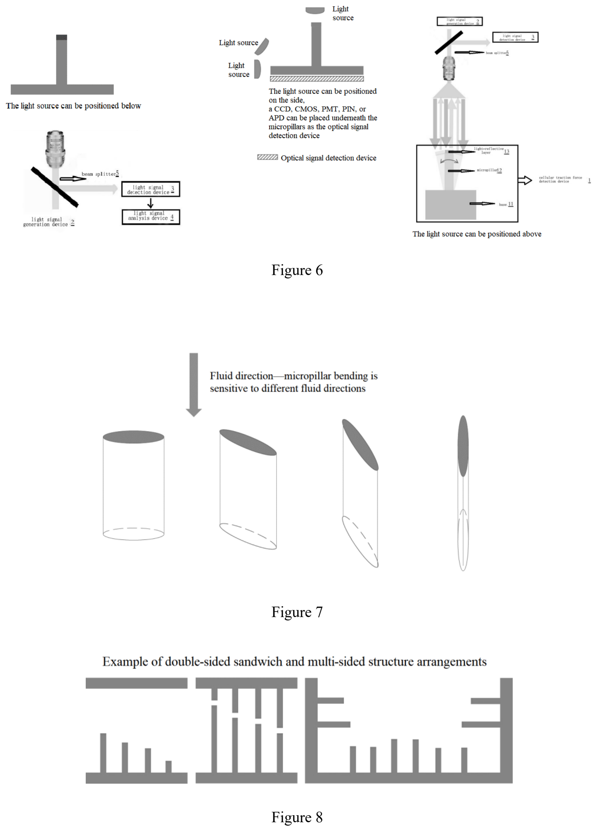

A multimodal fluid characterization and perturbation system is composed of one of the above multimodal fluid characterization and perturbation apparatuses 1, a light signal emitting device 2, a light signal detecting device 3, and a magnetic field emitting device 4. Wherein, the light signal emitting device 2 is used to emit a predetermined light beam, the light signal detecting device 3 is used to detect the light beam reflected from the light-reflecting layer 13, and the magnetic field emitting device 4 is used to emit a magnetic field to generate a magnetic force with the magnetic metal. The light beam emitted by the light signal emitting device 2 illuminates the light-reflecting layer 13 through an incident light path, and the light beam reflected by the light-reflecting layer 13 enters the light signal detecting device 3 through a reflected light path (Figure 6).

When using the above multimodal fluid characterization and perturbation system to characterize a fluid, the specific operational steps are as follows:

S1. Pass the fluid to be tested (characterized) through the multimodal fluid characterization and perturbation apparatus.

S2. Emit a predetermined light beam with the light signal emitting device.

S3. Detect the light beam after it has interacted with the multimodal fluid characterization and perturbation apparatus with the light signal detecting device. The "interaction" in this embodiment may only be the reflection effect on the predetermined light beam produced by the light-reflecting layer provided at any one part of the top end or side surface of the micropillars of the multimodal fluid characterization and perturbation apparatus, or it may be the reflection effect on the predetermined light beam produced by the light-reflecting layers provided on both the top end and the side surface of the micropillars. Preferably, it may also be that, under the action of a magnetic force from a magnetic field of a specific direction and intensity emitted by the magnetic field emitting device, the magnetic metal light-reflecting layer provided on the top end of the micropillar causes the micropillar to tilt in one direction, deform in the fluid, swing, or be pulled by magnetic force. Therefore, using the system of this embodiment, the state of the fluid can be characterized alone, or the flow can be perturbed alone, or the fluid can be characterized or perturbed simultaneously or alternately.

It should be pointed out that the fluid to be characterized in this embodiment can be static or flowing.

**Embodiment 2**

**A fabrication method for a multimodal fluid characterization apparatus**

This embodiment provides a fabrication method for a multimodal fluid characterization apparatus, which achieves the reflection of an optical signal by coating a metal reflective layer on the top end of micro-nano pillars.

S1. Spin-coat a layer of photoresist on the micro-nano pillar array. This step can protect the side surfaces of the micro-nano pillars and avoid metal deposition during subsequent processing.

S2. Use oxygen plasma etching technology to expose the top ends of the micro-nano pillars. This step ensures that the metal coating only covers the top ends of the micro-nano pillars and does not affect the side surfaces.

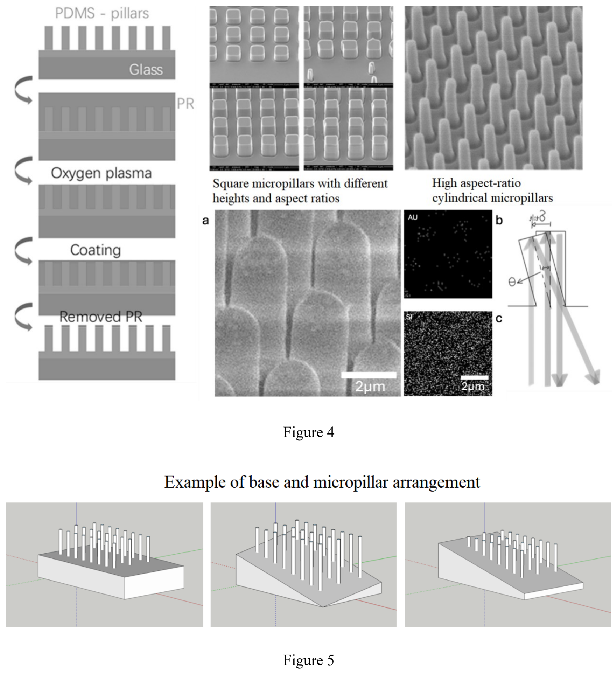

S3. Deposit a metal coating using an electron beam evaporator. This step will form a metal reflective layer on the exposed top ends of the micro-nano pillars, achieving the reflection of an optical signal. If a magnetic metal is used, a magnetic metal reflective layer can be formed on the top ends of the micro-nano pillars, achieving both the reflection of an optical signal and a response to a magnetic field. This can also be repeated multiple times to achieve the deposition of different metals or coatings with different functions (Figure 4).

S4. Remove the photoresist. This step can clear the residual photoresist, ensuring that the metal layer only covers the top ends of the nano-pillars. And use a scanning electron microscope to characterize the fabricated chip samples and confirm the successful setting of the metal coating (Figure 4).

Through the above preparation process, we can obtain a micropillar chip with a metal coating or a magnetic metal top, used for the reflection and detection of an optical signal, as well as for achieving a response to a magnetic field.

**Embodiment 3**

**A conversion method between micropillar deformation and the force applied**

This embodiment provides a conversion method between deformation and the force applied, based on the deformation of the micropillar and Hooke's law formula, and considering the effects of the deformation at the bottom ends of the micropillar.

S1. Calculate the ideal spring constant of the micropillar, k_bend:

$$k\_bend = (3/64) * \pi * E * D^4 / L^3$$

where E is Young's modulus, D is the diameter of the micropillar, and L is the length of the micropillar.

S2. Calculate the tilting coefficient, T_tilt(v):

$$T\_tilt(v) = a * (1 + v) / (2 * \pi) * \{2 * (1 - v) + (1 - 1 / (4 * (1 - v)))\}$$

where v is Poisson's ratio, and a is a correction coefficient.

S3. Calculate the deformation proportionality factor, a:

$$a = \delta\_bend / (\delta\_bend + \delta\_shear + \delta\_tilt)$$

where $\delta$_bend is the bending deformation of the micropillar, $\delta$_shear is the shear deformation, and $\delta$_tilt is the tilting deformation at the bottom end of the micropillar.

S4. Calculate the corrected spring constant, k_corr:

$$k\_corr = k\_bend \times a$$

S5. Calculate the force applied on the micropillar, F:

$$F = k\_corr \times \delta$$

where $\delta$ is the deformation of the micropillar.

S6. Calculate the deformation at the top end of the micropillar, $\delta$_tilt (Figure 4):

$$\delta\_tilt = 8 * T\_tilt(v) * (L/D)^2 * (4/\pi) * F / (E*D)$$

Through the above calculation method, we can consider the effects of the deformation at the bottom end of the micropillar and calculate the force applied on the micropillar more accurately. This method can be widely applied in fields such as microfluidics, micro-control, microreactors, chemical engineering, and biological fluids, providing an effective way to calculate the force applied on micropillars.

**Embodiment 4**

**Measuring Fluid Viscosity Using a Multimodal Fluid Characterization and Perturbation apparatus**

This embodiment provides a multimodal fluid characterization and perturbation apparatus that utilizes changes in the light reflection signal from a micropillar to reflect the micropillar's deflection and reset speeds, as well as frequency changes, under the action of a magnetic field, thereby achieving precise measurement of fluid viscosity.

When using the aforementioned multimodal characterization and perturbation system to measure fluid viscosity characteristics, the specific operational steps are as follows:

**S1.** Prepare a set of fluid samples with different viscosities, such as water, glycerol, and olive oil.

**S2.** Place the multimodal fluid characterization and perturbation apparatus in a stable experimental environment and connect it to a magnetic field generating device, a light signal emitting device, and a light signal detection device.

**S3.** Pass the fluid sample to be measured through the multimodal fluid characterization and perturbation apparatus.

**S4.** Use the magnetic field generating device to apply a magnetic field to the micropillars in the flow field, causing the micropillars to deflect. The change in the intensity of the micropillar's light reflection signal over time reflects its corresponding deflection speed in fluids of different viscosities. After the magnetic field is turned off, the change in the intensity of the light reflection signal over time reflects its reset speed and frequency in fluids of different viscosities (Figure 1).

**S5.** Light emitted from the light signal emitting device illuminates the reflective layer on the top end and/or side surface of the micropillar. Before and after this process of turning the magnetic field on and off, use the light signal detection device to detect the light reflected from the reflective layer on the top end and/or side surface of the micropillar. Analyze the collected optical signal data to extract features such as the micropillar's deflection speed, reset speed, and frequency information in fluids of different viscosities.

**S6.** Use the obtained data to plot graphs of the relationship between viscosity and deflection speed, reset speed, and frequency. Through these relationship graphs, the viscosity of an unknown fluid sample can be accurately evaluated.

This method can be widely applied in fields such as microfluidics, microreactors, chemical engineering, and biofluids, improving the accuracy and efficiency of fluid state monitoring.

**Embodiment 5**

**Measuring Fluid Status Across Different Flow Rate Ranges Using a Multimodal Fluid Characterization and Perturbation** apparatus

This embodiment provides a method for extending the applicable range of fluid status measurement by using a multimodal fluid characterization and perturbation device with an array of micropillars of different lengths and stiffnesses (Figure 5).

When using the aforementioned multimodal fluid characterization and perturbation system to characterize a fluid, the specific operational steps are as follows:

**S1.** Prepare a set of fluid samples with different viscosities and flow rates.

**S2.** Design and fabricate a set of micropillar arrays with different lengths and stiffnesses. The stiffness of these micropillars can be adjusted by changing the material or cross-linking strength to obtain a set of micropillars with varying stiffness to meet the needs of different fluid status measurements.

**S3.** Place the multimodal fluid characterization and perturbation apparatus in a stable experimental environment and connect it to a light signal emitting device and a light signal detection device.

**S4.** Pass the fluid samples to be measured one by one through the multimodal fluid characterization and perturbation apparatus .

**S5.** Use the light signal emitting device to emit a predetermined light beam to illuminate the reflective layer on the top end and/or side surface of the micropillars.

**S6.** Use the light signal detection device to detect the light reflected from the reflective layer on the top end and/or side surface of the micropillars. By analyzing the reflected light data, determine which micropillars are within the sensitive linear range and convert the linear deformation into an optical reflection signal.

**S7.** Based on the obtained data, plot graphs of the relationship between fluid flow rate and micropillar deformation and the optical reflection signal. Through these relationship graphs, the status of the fluid to be measured can be accurately evaluated.

Through this embodiment, we can expand the applicable measurement range.

**Embodiment 6**

**Measuring Fluid Direction Using Micropillars with Specific Cross-Section Shapes**

This embodiment provides a method for accurately measuring fluid direction by observing light attenuation using an array of micropillars with elliptical cross-sections and varying axial orientations.

When using the aforementioned multimodal fluid characterization and perturbation system to characterize a fluid, the specific operational steps are as follows:

**S1.** Prepare a set of fluid samples.

**S2.** Design and fabricate a set of micropillar arrays with elliptical cross-sections and varying axial orientations (Figure 7). These micropillars have different deformation characteristics along different axes to meet the needs of fluid direction measurement.

**S3.** Place the multimodal fluid characterization and perturbation apparatus in a stable experimental environment and connect it to a light signal emitting device and a light signal detection device.

**S4.** Pass the fluid samples to be measured one by one through the multimodal fluid characterization and perturbation apparatus.

**S5.** Use the light signal emitting device to emit a predetermined light beam to illuminate the reflective layer on the top end and/or side surface of the elliptical cross-section micropillars.

**S6.** Use the light signal detection device to detect the light reflected from the reflective layer on the top end and/or side surface of the elliptical cross-section micropillars. By analyzing the reflected light data, observe the deformation and optical reflection signals of micropillars with different axial orientations under different fluid directions.

**S7.** Analyze the collected optical signal data to extract the deformation and optical reflection signal characteristics of the elliptical cross-section micropillars with different axial orientations under different fluid directions.

**S8.** Based on the obtained data, plot graphs of the relationship between fluid direction and the deformation and optical reflection signals of the elliptical cross-section micropillars. Through these relationship graphs, the direction of the fluid to be measured can be accurately evaluated.

Through this embodiment, we can accurately measure fluid direction, providing strong support for fluid dynamics research.

**Embodiment 7**

**Three-Dimensional Analysis of Fluid Status at Different Layers Using Micropillars of Varying Positions, Lengths, and Aspect Ratios**

This embodiment provides a method for achieving 3D profiling of the status of different fluid layers by arranging micropillars with different lengths and aspect ratios at various positions within a three-dimensional fluid space, thereby enabling three-dimensional analysis of the fluid within an optimal measurement range.

When using the aforementioned multimodal fluid characterization and perturbation system to characterize a fluid, the specific operational steps are as follows:

**S1.** Prepare a set of different fluid samples.

**S2.** Design and fabricate a set of micropillars with different lengths and aspect ratios (Figure 8). These micropillars are arranged at different positions within the three-dimensional fluid space to meet the needs of 3D profiling.

**S3.** Place the multimodal fluid characterization and perturbation apparatus in a stable experimental environment and connect it to a light signal emitting device and a light signal detection device.

**S4.** Pass the fluid samples to be measured one by one through the multimodal fluid characterization and perturbation apparatus .

**S5.** Use the light signal emitting device to emit a predetermined light beam to illuminate the reflective layer on the top end and/or side surface of the micropillars with different lengths and aspect ratios.

**S6.** Use the light signal detection device to detect the light reflected from the reflective layer on the top end and/or side surface of the micropillars with different lengths and aspect ratios.

**S7.** Analyze the collected optical signal data to extract information on the deformation and optical reflection signals of the micropillars at different positions under the action of the fluid.

**S8.** Based on the obtained data, plot graphs of the status of different fluid layers and map into a three-dimensional space. Through these 3D profiling maps, the flow field status of the fluid to be measured in three-dimensional space can be accurately evaluated.

Through this embodiment, we can accurately achieve 3D profiling of the status of different fluid layers, providing strong support for fluid dynamics research.

**Embodiment 8**

**Monitoring Fluid Pressure by Characterizing Base Deformation Using Optical Reflection Signals**

This embodiment provides a method that utilizes the deformability of a base to infer the base's deformation and the pressure at that point by monitoring the attenuation of optical signals at different positions.
When using the aforementioned multimodal fluid characterization and perturbation system to characterize a fluid, the specific operational steps are as follows:

**S1.** Prepare a set of fluids with different pressures.

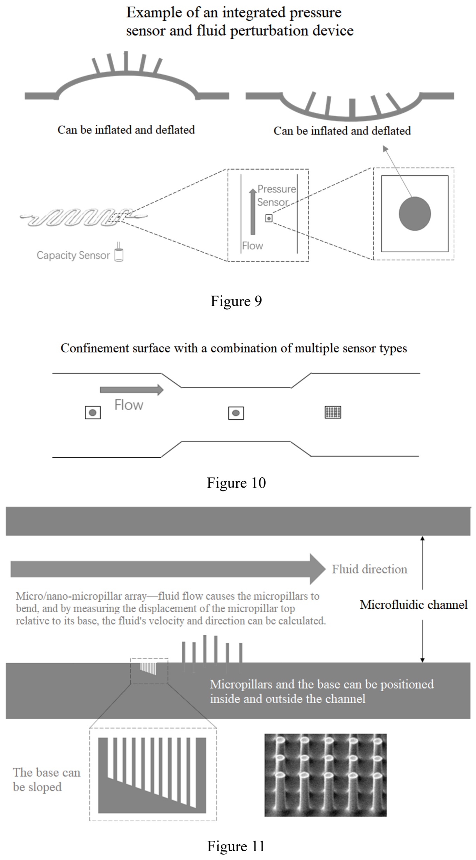

**S2.** Design and fabricate a base made of a flexible material with a hollow channel underneath, allowing the base to deform more freely into a curved surface under fluid pressure. Micropillars with a light-reflective layer are arranged on top of the base (Figure 9).

**S3.** Place the multimodal fluid characterization and perturbation apparatus in a stable experimental environment and connect it to a light signal emitting device, a light signal detection device, and a magnetic field generating device.

**S4.** Pass the fluids to be measured one by one through the multimodal fluid characterization and perturbation apparatus .

**S5.** Use the light signal emitting device to emit a predetermined light beam to illuminate the reflective layer on the top end and/or side surface of the micropillars on the base, which has been deformed by fluid pressure.

**S6.** Use the light signal detection device to detect the light reflected from the reflective layer on the top end and/or side surface of the micropillars arranged on the base that has been deformed by fluid pressure. By analyzing the reflected light data, observe the deformation of the base and the optical reflection signals under different fluid pressures.

**S7.** Analyze the collected optical signal data to extract the corresponding relationship between the deformation state of the base and the optical reflection signal information under different fluid pressures.

**S8.** Based on the obtained data, infer the deformation of the base and the pressure at that point.

Through this embodiment, we can accurately monitor fluid pressure, providing strong support for fluid mechanics research.

**Embodiment 9**

**Characterizing Fluid Parameters by Arranging Multimodal Fluid Characterization** apparatuses **at Different Positions**

This embodiment aims to provide a method for measuring the pressure difference between two points in a fluid with varying pipe diameters using multiple multimodal fluid characterization systems, and to calculate features such as the flow velocity and pressure at the center of the pipe based on the known pipe diameter, distance between the two points, and liquid viscosity.

**S1.** Configure confinement surfaces to change the pipe diameter at different locations and set up multiple multimodal fluid characterization systems to measure the pressure difference between two points in the fluid with varying pipe diameters (Figure 10).

**S2.** Collect known conditions, including the pipe diameter (D) and the distance between the two points (L). Use the multimodal fluid characterization system to measure the liquid viscosity ($\mu$) as described in Embodiment 4.

**S3.** Use the multimodal fluid characterization system to measure the pressure difference ($\Delta P$) between the two points in the fluid with varying pipe diameters.

**S4.** Calculate the flow velocity at the center of the pipe (V_max) based on the known conditions. Apply the Hagen-Poiseuille Equation: Vmax=4$\mu$LR2$\Delta$P, where R is the pipe radius, to obtain the central flow velocity.**S5.** Further calculate the flow rate (Q) of the pipe. The flow rate Q can be obtained by integrating the velocity (V) over the pipe's cross-section. For laminar flow, the velocity distribution along the radius is parabolic. The flow rate for laminar flow can be calculated using the following formula:

$$Q = (\pi R^4 \triangle P) / (8 \mu L).$$

.

This embodiment provides a method based on a multimodal fluid characterization system to measure the pressure difference between two points in a fluid with varying pipe diameters, and to calculate the central flow velocity, pressure, and subsequently the flow rate, based on known pipe diameter, distance, and liquid viscosity. This method facilitates the analysis of pressure, velocity, and flow rate distributions in fluids with varying pipe diameters.

**Embodiment 10**

**Achieving Fluid Perturbation through Base Deformation and Magnetic Control of Micropillar Movement**

This embodiment provides a method for perturbing a fluid, such as for mixing, by inducing base deformation through a mechanical device or by controlling micropillar movement with a magnetic field.
When using the aforementioned multimodal characterization and perturbation system to simultaneously or alternately characterize and actively perturb a fluid, the specific operational steps are as follows:

**S1.** Prepare a set of fluid samples to be mixed, for example, liquid A and liquid B.

**S2.** Design and fabricate a multimodal fluid characterization and perturbation system with a micropillar structure, including a magnetic field generating device and a mechanical device.

**S3.** Mix the fluid samples A and B and pass them through the multimodal fluid characterization and perturbation apparatus.

**S4.** Operate the mechanical device to cause deformation of the base (Figure 9). The base deformation will generate disturbances within the fluid, thereby causing mixing.

**S5.** Simultaneously or alternately, operate the magnetic field generating device to emit a magnetic field of a predetermined direction and strength. The magnetic field acts on the micropillars, inducing their movement. The movement of the micropillars further enhances the fluid perturbation, promoting mixing.

**S6.** Use the light signal detection device to monitor the fluid status in real-time, analyzing the effectiveness of the fluid mixing based on the reflected light data from micropillars at different locations.

Through this embodiment, we can achieve effective perturbation of fluids, such as mixing. Simultaneously or alternately, the fluid status can also be characterized. This method can be widely applied in fields such as microfluidics, microreactors, chemical engineering, and biofluids, improving the effectiveness and efficiency of fluid mixing.

**Embodiment 11**

**Measuring Fluid Density by Inducing Micropillar Deformation with a Magnetic Field**

This embodiment provides a method for measuring fluid density, the core of which is the attenuation of an optical signal caused by the deformation of micropillars induced by a magnetic field. Under a magnetic field of the same strength, micropillars in a denser fluid will deform less due to the buoyant force acting on the center of mass, resulting in smaller optical signal attenuation. This allows for the calculation of the fluid's density.

When using the aforementioned multimodal fluid characterization and perturbation system to characterize a fluid, the specific operational steps are as follows:

**S1.** Prepare a set of fluid samples with different densities to be measured.

**S2.** Design and fabricate a multimodal fluid characterization and perturbation system with a micropillar structure, including a magnetic field generating device.

**S3.** Pass the fluid samples to be measured one by one through the multimodal fluid characterization and perturbation apparatus .

**S4.** Apply a magnetic field of the same strength to each fluid sample and observe the deformation of the micropillars under the magnetic field's action.

**S5.** Use the light signal detection device to monitor in real-time the optical signal attenuation caused by the deformation of the micropillars in each fluid sample.

**S6.** Analyze and compare the optical signal attenuation data for each fluid sample. Based on the attenuation data, confirm that micropillars in denser fluids deform less under the magnetic field, resulting in smaller optical signal attenuation.

**S7.** Calculate the density of the fluid samples by comparing the optical signal attenuation data using a data analysis unit.

This method can be widely applied in fields such as microfluidics, microreactors, chemical engineering, and biofluids, providing a simple and efficient way to measure fluid density.

**Embodiment 12**

**A Micropillar Array for Measuring Fluid or Gas Status with Dynamic Range Adjustment**

This embodiment provides a method for measuring the status of a fluid or gas (flow rate, direction, viscosity) primarily using micropillar technology, with the ability to dynamically extend the measurement range as needed.

When using the aforementioned multimodal characterization and perturbation system to characterize a fluid, the specific operational steps are as follows:

**S1.** Coat the top ends of PDMS micropillars with a magnetic metal reflective layer, and apply an anti-reflective coating between and on the side surfaces of the micropillars.

**S2.** Place the micropillar chip at the bottom of a microfluidic channel.

**S3.** Inject fluid at different flow rates into the microfluidic channel inlet. Illuminate the chip from below the device with a laser or visible light, and simultaneously measure the optical reflection signal generated from the top ends of the micropillars from below the device. As the flow rate increases, the bending of the micropillars increases, and the optical reflection signal weakens. Experiments have verified a linear relationship between flow rate and the optical reflection signal, thus the fluid status can be inferred from the intensity of the optical reflection signal generated by the micropillar deformation.

**S4.** When the flow rate is too fast and exceeds the linear measurement range, apply a magnetic field to exert an upward pull on the micropillars, which is equivalent to increasing the stiffness of the micropillars, thereby extending the measurement range.

This method can be widely applied in fields such as microfluidics, microreactors, chemical engineering, and biofluids, providing a simple and efficient way for fluid characterization and measurement.

**Embodiment 13**

**A Technical Method for Simulating a Vascular Environment by Combining Microfluidics and a Micropillar Chip**

This embodiment provides a method for simulating a vascular environment by combining microfluidics and a micropillar chip to study the interactions among cells and their effects on the fluid state.

**S1.** Place micropillar chips at the bottom of the inlet and outlet of the microfluidic channel to monitor the status of the incoming and outgoing fluid. The micropillars have a metal reflective coating on top ends, and an anti-reflective coating is applied on the side surfaces of and between the micropillars. The fluid flow rate is calculated from the intensity of the optical reflection signal.

**S2.** Pave the bottom of the microfluidic channel with a commercial micropillar cell mechanics chip, which can characterize the magnitude of cellular forces through micropillar deformation.

**S3.** Inject vascular endothelial cells into the microfluidic channel, allowing them to grow until they form a complete monolayer on the mechanics chip.

**S4.** Introduce immune cells or tumor cells into the microfluidic channel to interact with the endothelial cells on the chip. Observe the changes in the cell mechanics of the endothelial cells.

**Embodiment 14**

**A Technical Method for Simulating a Vascular Environment by Combining Microfluidics and a Micropillar Chip**

This embodiment provides a method for simulating a vascular environment by combining microfluidics and a micropillar chip to study the interactions among cells and their effects on the fluid state.

**S1.** Place micropillar chips at the bottom of the inlet and outlet of the microfluidic channel to monitor the status of the incoming and outgoing fluid. The micropillars have a metal reflective coating on top ends, and an anti-reflective coating is applied on the side surfaces of and between the micropillars. The fluid flow rate is calculated from the intensity of the optical reflection signal.

**S2.** Pave the bottom of the microfluidic channel with a commercial micropillar cell mechanics chip, which can characterize the magnitude of cellular forces through micropillar deformation.

**S3.** Inject vascular endothelial cells into the microfluidic channel, allowing them to grow until they form a complete monolayer on the mechanics chip.

**S4.** Introduce immune cells or tumor cells into the microfluidic channel to interact with the endothelial cells on the chip. Observe the changes in the cell mechanics of the endothelial cells.

**Embodiment 15**

**Measuring Flow Velocity Distribution and Flow Rate in a Pipe Using a Multimodal Fluid Characterization System**

This embodiment aims to provide a method for measuring the distribution of fluid velocity and the flow rate within a pipe using a multimodal fluid characterization system.

**S1.** Arrange the multimodal fluid characterization system on the pipe wall and inside the pipe. Collect known conditions including the pipe diameter (D). Measure the liquid viscosity ($\mu$) and the shear stress ($\tau$) at the pipe wall (Figure 11).

**S2.** Determine the radial position (r) within the pipe, where $0 \leq r < R$, and R is the pipe radius.

**S3.** Apply the Newtonian fluid model, where shear stress $\tau$ is proportional to the velocity gradient (dv/dr): $\tau = \mu(dv/dr)$.

**S4.** Express the velocity gradient (dv/dr) in terms of shear stress $\tau$ and viscosity $\mu$: $dv/dr = \tau/\mu$.

**S5.** Integrate the velocity gradient (dv/dr) to obtain the velocity distribution formula: $V(r) = (\tau/\mu) \cdot (R-r) + C$, where C is the integration constant.

**S6.** At the center of the pipe (r = 0), the flow velocity is at its maximum, denoted as V_max. Substitute this into the velocity distribution formula to solve for the constant C: $Vmax = (\tau/\mu) \cdot R + C$, which gives $C = Vmax - (\tau/\mu) \cdot R$.

**S7.** Substitute the constant C back into the velocity distribution formula to get the final velocity distribution formula: $V(r) = (\tau/\mu) \cdot (R-r) + Vmax - (\tau/\mu) \cdot R$.

**S8.** Based on the velocity distribution formula, calculate the velocity values at different radial positions (r) to obtain the velocity distribution within the pipe.

**S9.** Using the velocity distribution formula and the pipe's cross-sectional area ($A = \pi R2$), calculate the flow rate (Q) of the fluid in the pipe: $Q = \int 0^R V(r) \cdot 2\pi rdr - 2\pi \int 0^R [\tau/\mu \cdot (R-r) + Vmax - (\tau/\mu \cdot R] \cdot rdr$.

This embodiment provides a method for measuring the flow velocity distribution and flow rate within a pipe using a multimodal fluid characterization system. This method facilitates the analysis of velocity distribution at different radial positions within the pipe, helping to understand the fluid's flow characteristics. Furthermore, the method can also be used to calculate the total flow rate of the fluid in the pipe.

**Embodiment 16**

**Measuring a Flow Field and Performing Flow Velocity Calibration Using Reflected Light from Micropillar Sensors**

This embodiment aims to provide a method for accurately inferring fluid status by measuring a flow field and utilizing flow velocity calibration through the reflected light from micropillar sensors.

**S1.** The top ends of the PDMS micropillars have a metal reflective coating, and an anti-reflective coating is applied between and on the side surfaces of the micropillars.

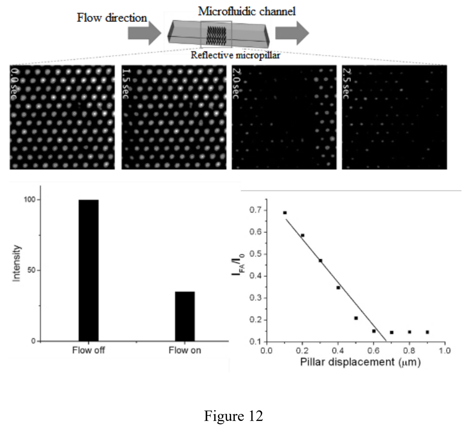

**S2.** This micropillar chip is placed at the bottom of a microfluidic channel (Figure 12).

S3. Fluid is injected into the microfluidic channel inlet at different flow rates. A laser or visible light is shone onto the chip from below the device, and simultaneously, the optical reflection signal generated from the top ends of the micropillars is measured from below the device. As the flow rate increases, the bending of the micropillars increases, and the optical reflection signal weakens. It has been experimentally confirmed that within a certain deformation range, the flow rate and the optical reflection signal show a linear relationship. Therefore, the fluid status can be inferred from the intensity of the optical reflection signal generated by the micropillar deformation.

This embodiment provides a method for measuring a flow field and performing flow velocity calibration using the reflected light from micropillar sensors. Within a certain deformation range, the deformation and the attenuation of the optical reflection signal are linearly related, allowing the fluid status to be inferred from the intensity of the optical reflection signal generated by the micropillar deformation.

Finally, it should be noted that the above embodiments are merely for illustrating the technical solutions of the present invention and not for limiting it. Although the present invention has been described in detail with reference to the foregoing embodiments, a person of ordinary skill in the art should understand that they can still modify the technical solutions described in the foregoing embodiments or make equivalent substitutions for some or all of the technical features therein. Such modifications or substitutions do not deviate from the essence of the corresponding technical solutions from the scope of the technical solutions of the embodiments of the present invention.

**Claims**

1. A multimodal fluid characterization and perturbation apparatus, **characterized in that** it comprises:

   a base, and
   a micropillar array arranged on the base, the micropillar array comprises at least one micropillar, the micropillar is arranged to deform under the action of a fluid or a magnetic force;
   the micropillar comprises a bottom end connected to the base, a side surface, and a top end opposite the bottom end and away from the base, the micropillar being provided with a light-reflecting layer, the light-reflecting layer being deployed at least at one position of the top end, side surface, and within the pillar body of the micropillar.

2. The multimodal fluid characterization and perturbation apparatus according to claim 1, **characterized in that** an anti-reflection layer is provided on the side surface of the micropillar and/or the surface of the base.

3. The multimodal fluid characterization and perturbation apparatus according to claim 1, **characterized in that** the light-reflecting layer is selected from at least one of metals, metal oxides, metal salts, ultrafine glass beads, microprisms, and organic reflective materials.

4. The multimodal fluid characterization and perturbation apparatus according to claim 1, **characterized in that** a magnetic substance is disposed at least one position at the top end, side surface, and/or within the pillar body of the micropillar; wherein preferably, a magnetic metal light-reflecting layer is arranged at the top end of the micropillar.

5. The multimodal fluid characterization and perturbation apparatus according to claim 1, **characterized in that** it further comprises several flow field confinement structures arranged on the base, the flow field confinement structure comprising one or several confinement surfaces, wherein the confinement surface being a plane or a curved surface that is perpendicular to the plane where the base is located, and connected to the base or integrally formed with the base.

6. The multimodal fluid characterization and perturbation apparatus according to claim 1, **characterized in that** the base and/or micropillars are made of a conductive material.

7. The multimodal fluid characterization and perturbation apparatus according to claim 1, **characterized in that** a fluorescent substance is provided on the extension part of the micropillar from the top end to the bottom end, preferably, the cross-section of the micropillar includes a circle, an ellipse, or a polygon.

8. A multimodal fluid characterization and perturbation characterization system, **characterized in that** it comprises:

   a multimodal fluid characterization and perturbation apparatus according to any one of claims 1-7;
   a light signal emitting device, for emitting a predetermined light beam;
   a light signal detecting device, for detecting the light beam reflected from the light-reflecting layer;
   a magnetic field emitting device, for emitting a magnetic field;
   wherein the light-reflecting layer is arranged to be illuminated, through an incident light path, by the light beam emitted by the light signal emitting device , and the light beam reflected by the light-reflecting layer enters the light signal detecting device through a reflected light path.

9. The multimodal fluid characterization and perturbation system according to claim 8, **characterized in that** it further comprises a light signal analysis device, the light signal analysis device being used to analyze the light signal.

10. The multimodal fluid characterization and perturbation system according to claim 9, **characterized in that** it further comprises a data processing device, for computing and processing the light signal to obtain a flow field state result; and/or,
directly obtaining the flow field state result from the light signal analyzed by the light signal analysis device.

11. The multimodal fluid characterization and perturbation system according to claim 10, **characterized in that** the data processing device assesses the chemical, physical, and biological states within the system based on the flow field state, and provides predictive reaction information.

12. The multimodal fluid characterization and perturbation characterization system according to claim 8, **characterized in that** it further comprises a pressure sensing device, for measuring pressure information and/or applying pressure to the fluid.

13. The multimodal fluid characterization and perturbation system according to claim 8, **characterized in that** the light signal emitting device comprises a light source, the light source comprises a first light source arranged at the bottom end of the base and/or a second light source arranged at the side surface of the base, wherein the light beam emitted by the first light source and/or second light source reaches the micropillars.

14. The multimodal fluid characterization and perturbation system according to claim 8, **characterized in that** it further comprises a motion or deformation device, wherein the device applies a mechanical force to the multimodal fluid characterization and perturbation system and causes motion and/or deformation.

15. The multimodal fluid characterization and perturbation system according to claim 8, **characterized in that** it consists of a double-sided structure or multi-sided structure comprises two or more multimodal fluid characterization and perturbation devices and wherein the outer sides of the double-sided or multi-sided structure are the bases, and the inner sides enclose a three-dimensional containment chamber for a fluid;
the base of each multimodal fluid characterization and perturbation apparatus forms a surface of the three-dimensional containment chamber; when forming the three-dimensional containment chamber, at least one surface of the three-dimensional containment chamber is connected with micropillars.

16. The multimodal fluid characterization and perturbation system according to claim 15, **characterized in that** the fluid confinement structures complements each other when two multimodal fluid characterization and perturbation devices form the double-sided or multi-sided structure.

17. A method for performing fluid characterization and perturbation using the multimodal fluid characterization and perturbation system according to any one of claims 8-16, **characterized in that** it comprises the following steps:

emitting a predetermined light beam using the light signal emitting device;
detecting the light beam after it has interacted with the multimodal fluid characterization and perturbation apparatus using the light signal detecting device.

18. The method according to claim 17, **characterized in that** it further comprises the step of emitting a magnetic field of a predetermined direction and intensity using the magnetic field emitting device.

19. The method according to claim 18, **characterized in that** it further comprises a light signal analysis step: using the light signal analysis device to perform a comparative analysis of the reflected light beams before and after the fluid to be characterized passes through the multimodal fluid characterization and perturbation apparatus,
and/or,

using the light signal analysis device to perform a comparative analysis of the reflected light beams before and after the magnetic field emitting device emits a magnetic field a predetermined direction and intensity, to obtain fluid information;
the fluid information comprising the flow field shear force, flow field direction, flow velocity, viscous force, and flow state at a certain specific location, and the dynamic changes over time of the spatial distribution of the fluid information.

20. Applications of the multimodal fluid characterization and perturbation apparatus and system according to any one of claims 8-16in microfluidics, micro-control, microreactors, chemical engineering, and biological fluids.

Figure 1

Example of magnetic material and light-reflective layer arrangement

The magnetic metallic material serves as the light-reflective layer

The magnetic material is positioned on top of the light-reflective layer

The magnetic material is positioned on the side of the micropillar, and preferably, the magnetic material can serve as an anti-reflective layer

Magnetic or superparamagnetic microbeads are dispersed within the micropillar

The light-reflective layer is in the middle of the micropillar

The light-reflective layer is at the bottom of the micropillar

Magnetic metallic substance        Magnetic substance        Light-reflective layer

Figure 2

Examples of fluorescent material and light-reflective layer arrangements

The fluorescent material fills the entire micropillar

The fluorescent material is positioned below the light-reflective layer

The fluorescent material is positioned on the side of the micropillar

The fluorescent material is dispersed within the micropillar

Magnetic metallic substance        Magnetic substance        Light-reflective layer

Figure 3

Figure 4

Example of base and micropillar arrangement

Figure 5

The light source can be positioned below

beam splitter 5

light signal detection device 3

light signal analysis device 4

light signal generation device 2

Light source

Light source

Light source

The light source can be positioned on the side, a CCD, CMOS, PMT, PIN, or APD can be placed underneath the micropillars as the optical signal detection device

▨▨ Optical signal detection device

light signal generation device 2

light signal detection device 3

beam splitter 5

light-reflective layer 13

micropillar 2

base 11

cellular traction force detection device 1

The light source can be positioned above

Figure 6

Fluid direction—micropillar bending is sensitive to different fluid directions

Figure 7

Example of double-sided sandwich and multi-sided structure arrangements

Figure 8

Example of an integrated pressure
sensor and fluid perturbation device

Can be inflated and deflated          Can be inflated and deflated

Figure 9

Confinement surface with a combination of multiple sensor types

Figure 10

Figure 11

Figure 12

## INTERNATIONAL SEARCH REPORT

| International application No. |
| --- |
| **PCT/CN2024/083360** |

### A. CLASSIFICATION OF SUBJECT MATTER

G01N33/543(2006.01)i; G01N11/00(2006.01)i; C12M1/42(2006.01)i; G01N21/55(2014.01)i

According to International Patent Classification (IPC) or to both national classification and IPC

### B. FIELDS SEARCHED

Minimum documentation searched (classification system followed by classification symbols)

IPC: G01N,C12M

Documentation searched other than minimum documentation to the extent that such documents are included in the fields searched

Electronic data base consulted during the international search (name of data base and, where practicable, search terms used)

CNTXT, DWPI, ENTXT, ENTXTC, VEN, WPABS, WPABSC, ISI web of Science, CNKI, Pubmed: 磁, 磁场, 反射, 光源, 光线 反射, 微柱, 阵列, 流体, 形变, 阻挡, 荧光, 压力, 顶端, 底部, 侧面, 运动, 申请人/发明人, magnatic, reflect+, light, micropost, array, flow, motion, fluorescent, pressure, top, bottom, move, side,

### C. DOCUMENTS CONSIDERED TO BE RELEVANT

| Category* | Citation of document, with indication, where appropriate, of the relevant passages | Relevant to claim No. |
| --- | --- | --- |
| PX | WO 2023046166 A1 (RUIXIN FUZHOU TECHNOLOGY CO., LTD.) 30 March 2023 (2023-03-30) claims 1-4, 9, and 11-16, and description, embodiments 10, 12, 14, and 18 | 1-3, 5, 8-11, 14-17, 20 |
| X | US 2015300953 A1 (THE UNIVERSITY OF NORTH CAROLINA AT CHAPEL HILL) 22 October 2015 (2015-10-22) claims 1, 36, 40, 42, 44, 63-64, 76, and 83, description, paragraphs 15, 19-28, 33-34, and 46-71, and figures 1-4 | 1-11, 14-20 |
| Y | US 2015300953 A1 (THE UNIVERSITY OF NORTH CAROLINA AT CHAPEL HILL) 22 October 2015 (2015-10-22) claims 1, 36, 40, 42, 44, 63-64, 76, and 83, description, paragraphs 15, 19-28, 33-34, and 46-71, and figures 1-4 | 12-14, 17-20 |
| Y | CN 111664976 A (BEIJING UNIVERSITY OF CHEMICAL TECHNOLOGY) 15 September 2020 (2020-09-15) claims 1-3 and 6-7 | 12-14, 17-20 |

✓ Further documents are listed in the continuation of Box C.    ✓ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **03 July 2024** | **09 July 2024** |

| Name and mailing address of the ISA/CN | Authorized officer |
| --- | --- |
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

# EP 4 692 795 A1

## INTERNATIONAL SEARCH REPORT

| International application No. |
| --- |
| **PCT/CN2024/083360** |

### C. DOCUMENTS CONSIDERED TO BE RELEVANT

| Category* | Citation of document, with indication, where appropriate, of the relevant passages | Relevant to claim No. |
| --- | --- | --- |
| X | US 2012156791 A1 (RICHARD SUPERFINE et al.) 21 June 2012 (2012-06-21) claims 1-10 and 12-15 | 1-11, 14-20 |
| Y | US 2012156791 A1 (RICHARD SUPERFINE et al.) 21 June 2012 (2012-06-21) claims 1-10 and 12-15 | 12-14, 17-20 |
| A | CN 111721451 A (BEIJING UNIVERSITY OF CHEMICAL TECHNOLOGY) 29 September 2020 (2020-09-29) claims 1 and 5, and description, paragraphs 19 and 46 | 1-20 |
| A | US 2013109012 A1 (UNIVERSITY OF WASHINGTON CENTER FOR COMMERCIALIZ) 02 May 2013 (2013-05-02) claims 16-19 | 1-20 |
| A | US 2018372635 A1 (THE REGENTS OF THE UNIVERSITY OF CALIFORNIA) 27 December 2018 (2018-12-27) claims 1-40 | 1-20 |
| A | CN 109416312 A (RHEOMICS INC.) 01 March 2019 (2019-03-01) claims 1-83 | 1-20 |
| A | US 2019139688 A1 (THE BOARD OF TRUSTEES OF THE UNIVERSITY OF ILLINOIS) 09 May 2019 (2019-05-09) description, paragraphs 87 and 88 | 1-20 |

**INTERNATIONAL SEARCH REPORT**
Information on patent family members

International application No.

**PCT/CN2024/083360**

| Patent document cited in search report | | | Publication date (day/month/year) | Patent family member(s) | | | Publication date (day/month/year) |
|---|---|---|---|---|---|---|---|
| WO | 2023046166 | A1 | 30 March 2023 | AU | 2022351105 | A1 | 09 May 2024 |
| US | 2015300953 | A1 | 22 October 2015 | US | 9952149 | B2 | 24 April 2018 |
| | | | | WO | 2014085804 | A1 | 05 June 2014 |
| | | | | EP | 2926115 | A1 | 07 October 2015 |
| | | | | EP | 2926115 | A4 | 06 July 2016 |
| | | | | EP | 2926115 | B1 | 26 May 2021 |
| CN | 111664976 | A | 15 September 2020 | CN | 111664976 | B | 20 August 2021 |
| US | 2012156791 | A1 | 21 June 2012 | US | 8586368 | B2 | 19 November 2013 |
| | | | | ES | 2823456 | T3 | 07 May 2021 |
| | | | | WO | 2010151780 | A2 | 29 December 2010 |
| | | | | WO | 2010151780 | A3 | 21 April 2011 |
| | | | | EP | 2446259 | A2 | 02 May 2012 |
| | | | | EP | 2446259 | A4 | 01 April 2015 |
| | | | | EP | 2446259 | B1 | 05 August 2020 |
| | | | | US | 2016209313 | A1 | 21 July 2016 |
| | | | | US | 9612185 | B2 | 04 April 2017 |
| | | | | JP | 2012531595 | A | 10 December 2012 |
| | | | | JP | 5511949 | B2 | 04 June 2014 |
| | | | | US | 2014001146 | A1 | 02 January 2014 |
| | | | | US | 9238869 | B2 | 19 January 2016 |
| CN | 111721451 | A | 29 September 2020 | CN | 111721451 | B | 28 September 2021 |
| US | 2013109012 | A1 | 02 May 2013 | US | 9140684 | B2 | 22 September 2015 |
| | | | | US | 2015056643 | A1 | 26 February 2015 |
| | | | | US | 9213024 | B2 | 15 December 2015 |
| | | | | US | 2016024454 | A1 | 28 January 2016 |
| | | | | US | 10006900 | B2 | 26 June 2018 |
| US | 2018372635 | A1 | 27 December 2018 | WO | 2017015063 | A1 | 26 January 2017 |
| | | | | US | 10712271 | B2 | 14 July 2020 |
| CN | 109416312 | A | 01 March 2019 | CA | 2998812 | A1 | 23 March 2017 |
| | | | | WO | 2017049279 | A1 | 23 March 2017 |
| | | | | US | 2018266951 | A1 | 20 September 2018 |
| | | | | US | 10900896 | B2 | 26 January 2021 |
| | | | | AU | 2022201238 | A1 | 24 March 2022 |
| | | | | AU | 2022201238 | B2 | 18 April 2024 |
| | | | | EP | 3350569 | A1 | 25 July 2018 |
| | | | | EP | 3350569 | A4 | 11 September 2019 |
| | | | | EP | 3350569 | B1 | 19 July 2023 |
| | | | | AU | 2016324467 | A1 | 05 April 2018 |
| | | | | AU | 2016324467 | B2 | 02 December 2021 |
| | | | | EP | 4252898 | A2 | 04 October 2023 |
| | | | | EP | 4252898 | A3 | 29 November 2023 |
| | | | | CN | 109416312 | B | 11 November 2022 |
| US | 2019139688 | A1 | 09 May 2019 | US | 10796831 | B2 | 06 October 2020 |

Form PCT/ISA/210 (patent family annex) (July 2022)